(19) Europäisches Patentamt
European Patent Office
Office européen des brevets

(11) Publication number: **0 007 738**
**B1**

(12) **EUROPEAN PATENT SPECIFICATION**

(45) Date of publication of patent specification: **29.12.82**

(21) Application number: **79301351.7**

(22) Date of filing: **10.07.79**

(51) Int. Cl.[3]: **C 07 C 102/00,**
C 07 C 103/44,
C 07 C 93/14

(54) **Process for producing 2-amino-4-acylaminophenyl ether and 2,4-diaminophenyl ether.**

(30) Priority: **10.07.78 JP 84162/78**

(43) Date of publication of application:
**06.02.80 Bulletin 80/3**

(45) Publication of the grant of the patent:
**29.12.82 Bulletin 82/52**

(84) Designated Contracting States:
**AT BE CH DE FR GB IT NL SE**

(56) References cited:
**DE - A - 1 543 625**
**FR - A - 1 395 763**
**FR - A - 2 349 570**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Fujii, Yozo**
**5-55 Kasumicho**
**Nishinomiya (JP)**
Inventor: **Yamaguchi, Hiromichi**
**977 Toyosatocho**
**Higashiyodogawa-ku, Osaka (JP)**
Inventor: **Tada, Kazuhiro**
**398 Narayacho**
**Fushimi-ku, Kyoto (JP)**
Inventor: **Kaneoya, Tatsuo**
**3-31 Sakura 5-chome**
**Minoo (JP)**
Inventor: **Takata, Takeshi**
**2-40 Hirata 1-chome**
**Ibaraki (JP)**
Inventor: **Kotera, Norio**
**19-22 Nanamatsucho**
**Amagasaki (JP)**

(74) Representative: **Geering, Keith Edwin et al,**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 007 738 B1

Process for producing 2-amino-4-acylaminophenyl ether and 2,4-diaminophenyl ether

The present invention relates to a process for producing a 2-amino-4-acylaminophenyl ether from a 2,4-dinitrophenyl ether.

2-amino-4-acylaminophenyl ethers are important intermediates as coupling components for the production of azo disperse dyes and have been produced, as known, by reduction of a 2,4-dinitrophenyl ether to give the corresponding 2,4-diaminophenyl ether and subsequent acylation of the isolated 2,4-diaminophenyl ether with an acylating agent, the 2,4-dinitrophenyl ether being produced by condensation of 2,4-dinitrochlorobenzene with an alcohol in the presence of sodium carbonate or sodium hydroxide.

For reduction of the 2,4-dinitrophenyl ether, it is known, as disclosed in German Offenlegungsschrift No. 2435818, to subject the 2,4-dinitrophenyl ether to catalytic hydrogenation using as a solvent aniline or a substituted aniline. In this process, however, the solvent must be separated from the desired 2,4-diaminophenyl ether after completion of the hydrogenation, resulting in a high production cost. There is also known a reduction process comprising subjecting the 2,4-dinitrophenyl ether to catalytic hydrogenation in an aqueous medium (published Japanese Patent Application No. 15327/1978). In this process, the dinitro compound in its aqueous slurry must be fed into an autoclave kept under a pressure of about 10.2 to 17.0 atm. (gauge) using a pump, and therefore it is industrially disadvantageous because the production cost is high due to the special production equipment and because of poor productivity.

In the acylation of the 2,4-diaminophenyl ether, at least one of a 2-acylamino compound and a 2,4-bis(acylamino) compound has inevitably been produced in addition to the desired 4-acylamino compound, making it difficult to produce the desired 4-acylamino compound selectively with good yield.

For example, there are known an acylation process for the production of the 2-amino-4-acylaminophenyl ether comprising carrying out the acylation in a reaction solvent of water, a lower alcohol, an ester or a ketone, or a mixture of water and said organic solvent (German Offenlegungsschrift No. 1543625), and a process comprising contacting a mineral acid salt of said diamine with an acylating agent in an aqueous medium (published Japanese Patent Applications Nos. 88035/1975 and 73831/1977). In the former process, the undesirable production of the 2-acylamino compound is low, whereas the undesirable 2,4-bis(acylamino) compound is produced in large amount so that the desired 2-amino-4-acylaminophenyl ether is formed in yields of at most 75%; and in the latter process, the production of 2,4-bis(acylamino) compound is low, whereas the 2-acylamino compound is produced in large amounts, so that the yield of the desired 4-acylamino compound becomes less than 80% and moreover the separation of the desired compound and by-products becomes difficult.

In order to solve the above problems relating to the reduction of 2,4-dinitrophenyl ether and the acylation 2,4-diaminophenyl ether, the present inventors have studied and found that the reduction is carried out effectively with hydrogen in the presence of a catalyst using as a reduction solvent an N-substituted amide of a lower aliphatic acid or a mixed solvent containing the same to give the desired 2,4-diaminophenyl ether in good yield and purity; and further that the acylation is carried out effectively by using as an acylation solvent an N-substituted amide of a lower aliphatic acid or a mixed solvent containing the same, whereby the desired 2-amino-4-acylaminophenyl ether may be obtained with a high selectivity of 95% or more, while the by-production of the 2-acylamino compound is low and that of the 2,4-bis(acylamino) compound is as little as 5% or less, and still further that the resulting reduction mixture comprising the 2,4-diaminophenyl ether and above-said reduction solvent can be subjected as it is to the subsequent acylation without isolation of the resulting 2,4-diaminophenyl ether from the reduction mixture.

The present invention provides a process for producing a 2-amino-4-acylaminophenyl ether of the formula

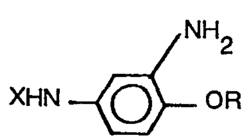

I

wherein R is a $C_1$—$C_9$ alkyl group, a halogen-, $C_1$—$C_3$ alkoxy-, phenyl- or phenoxy-substituted $C_1$—$C_4$ alkyl group or a phenyl group unsubstituted or substituted with one or two substituents selected from halogen atoms, $C_1$—$C_4$ alkyl groups, $C_1$—$C_4$ alkoxy groups and acylamino groups, and X is an acyl group, by contacting a 2,4-diaminophenyl ether of the formula,

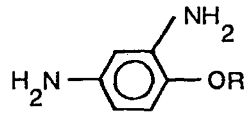 II

wherein R is as defined above, with an acylating agent in the presence of a solvent, characterized in that the solvent is selected from N-substituted amides of lower aliphatic acids and mixtures of such N-substituted acid amide with at least one other solvent selected from water, lower alcohols, lower carboxylic acids, ketones, and hydrocarbons, in which the amount of the other solvent is at most 40% by weight of the solvent mixture.

This invention also provides a process for producing a 2,4-diaminophenyl ether of the formula II above by catalytic hydrogenation of a 2,4-dinitrophenyl ether of the formula

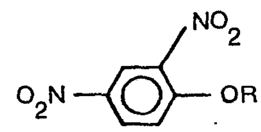

wherein R is as defined above in a solvent, characterized in that the solvent is selected from N-substituted amides of lower aliphatic acids and mixtures of such N-substituted acid amide with at least one other solvent selected from water, lower alcohols, lower carboxylic acids, ketones, and hydrocarbons, in which the amount of the other solvent is at most 30% by weight of the solvent mixture.

In the present specification, the term "acyl" includes acetyl, propionyl, benzoyl and the like, the term "halogen" includes chlorine and bromine, and examples of the substituted phenyl group include p-chlorophenyl, p-tolylphenyl, p-acetylaminophenyl, 2,4-dimethylphenyl and 2,4-dichlorophenyl.

Suitable N-substituted amides of lower aliphatic acids include N,N-dimethylformamide, N-methylformamide, N,N-dimethylacetamide, N-methylpropionamide and the like. Of these, N,N-dimethylformamide is advantageously used because it is economical and operations including isolation of the desired compound, if desired, and separation of the solvent after the reduction, if desired, are facilitated. Further, as the reduction solvent, there can also be used a mixture of such N-substituted acid amide and at least one solvent selected from water, lower alcohols (e.g. methyl alcohol, ethyl alcohol and isopropyl alcohol), lower carboxylic acids (e.g. formic acid, acetic acid and propionic acid), ketones and hydrocarbons (e.g. n-hexane, cyclohexane, benzene and toluene). The amount of the other solvent to be mixed with said N-substituted acid amide is at most 30% by weight of the mixture.

The amount of the reduction solvent is not critical, and varies depending on the reduction temperature. It is, however, preferred to dissolve the 2,4-dinitrophenyl ether completely during the hydrogenation. Usually, the amount is 0.5 to 10 times, preferably 1 to 5 times, the weight of the 2,4-dinitrophenyl ether. An amount greater than this may be used without any adverse affect on the reduction, but is uneconomical.

Metals usable for the catalyst in the present reduction are metals belonging to Group VIII of Mendelejeff's Periodic Table and include cobalt, nickel, platinum, palladium, osmium, iridium, ruthenium and rhodium. Of these, the preferred are platinum, palladium and nickel. The metal can be activated by a method known per se. The metal catalyst may be used as it is or may be supported on a carrier. The preferred are Raney nickel, platinum · carbon, palladium carbon and the like. The amount of the catalyst varies depending on the kind of catalyst. For example, the amount of platinum or palladium suitably used is 0.0005 to 0.1% by weight, preferably 0.001 to 0.05% by weight, more preferably 0.005 to 0.02% by weight, based on the weight of the 2,4-dinitrophenyl ethers; palladium-carbon or platinum-carbon may be used in an amount of 0.1 to 20% by weight, preferably 0.2 to 10% by weight, more preferably 0.5 to 3% by weight, based on the weight of the 2,4-dinitrophenyl ether. Raney nickel is usable in an amount of 0.1 to 20% by weight, preferably 0.2 to 10% by weight, more preferably 0.5 to 5% by weight, based on the weight of the 2,4-dinitrophenyl ether.

The reduction pressure and temperature are not particularly critical, and the reduction of the present invention can be effected even at room temperature under atmospheric pressure. However, in order to increase the reaction rate, raised temperature and/or increased pressure can be employed. Because the present reduction is exothermic, the temperature is controlled usually at 20° to 150°C., preferably at 50° to 100°C., while cooling the reaction system. In this respect, care should be taken because insufficient cooling of the reaction system may result in violent reaction.

As for the hydrogen pressure, there can be employed a hydrogen pressure of up to 100 Bars (gauge), preferably 5 to 40 Bars (gauge), more preferably 10 to 30 Bars (gauge).

The reaction time varies depending on various factors such as the kind and amount of the solvent and catalyst, the hydrogen pressure and the temperature. In general, the reaction is continued until

hydrogen is no longer absorbed. The completion of the reaction can be confirmed by a known method, for example, liquid chromatography.

The hydrogenation reaction in accordance with the present invention is suitably carried out as follows.

The 2,4-dinitrophenyl ether and the particular solvent and catalyst are placed in an appropriate reaction vessel under an inert gas atmosphere (i.e. nitrogen gas), and the mixture is adjusted to a desired temperature under a desired hydrogen pressure, while being thorughly mixed, for example, by stirring. After completion of the reaction, the reduction mixture can be, if desired, treated in a known manner such as distillation and crystallization, to obtain the desired 2,4-diaminophenyl ether.

In carrying out the acylation of the present invention, it is essential to use as an acylation solvent an N-substituted acid amide as defined above or a mixture thereof with at least one solvent selected from water, lower alcohols (e.g. methyl alcohol, ethyl alcohol and isopropyl alcohol), lower carboxylic acid (e.g. formic acid, acetic acid and propionic acid), ketones (e.g. acetone, methyl ethyl ketone and methyl isobutyl ketone) and hydrocarbons (e.g. n-hexane, cyclohexane, benzene and toluene). The amount of the other solvent to be mixed with the N-substituted acid amide is at most 40% by weight of the mixture.

The amount of the acylation solvent is not particularly critical but is is usually 2 to 15 times, preferably 3 to 10 times, the weight of the starting 2,4-diaminophenyl ether. The reaction solvent may be used in an amount more greater than this without any adverse effect on the acylation, but this is uneconomical.

The acylating agents usable in the acylation of the present invention include for example, aliphatic acid anhydrides (e.g. acetic anhydride, propionic anhydride), aromatic acid anhydrides (e.g. benzoic anhydride), and their acyl halides (e.g. acetyl chloride or bromide, benzoyl chloride or bromide).

The amount of the acylating agent is usually 0.6 to 1.05 mole, preferably 0.8 to 1.03 mole, per mole of the 2,4-diaminophenyl ether.

In this acylation, the starting 2,4-diaminophenyl ether may be any one produced in a manner known per se. However, the aforesaid reduction mixture can be applied to the present acylation as it is or if necessary after adjusting the amount of the N-substituted acid amide or the mixture containing the same as the solvent, whereby the desired 2-amino-4-acylaminophenyl ether can be obtained industrially advantageously in good yield with high selectivity.

In carrying out the acylation of the present invention, the starting 2,4-diaminophenyl ether can be dissolved in the reaction solvent of the N-substituted acid amide or the mixture containing the same, under an atmosphere of an inert gas (e.g. nitrogen gas) and then the acylating agent added thereto while the reaction system is stirred.

The acylation is carried out preferably at a relatively low temperature, usually at −10° to 40°C., favorably at 0° to 25°C., more favorably at 0° to 20°C.

After completion of the acylation, the desired 2-amino-4-acylaminophenyl ether product is isolated from the reaction mixture. For example, the acylation solvent and low boiling material are distilled off under reduced pressure to obtain the crude product, which can be, if desired, purified by further distillation under reduced pressure, or the like. Alternatively, a part of the acylation solvent and low boiling material is eliminated by distillation under reduced pressure to obtain a concentrate, and then water is added to the concentrate to precipitate the desired 2-amino-4-acylaminophenyl ether, which is separated by filtration and then dried.

The thus obtained 2-amino-4-acylaminophenyl ether is suitable for use as a starting material for the production of azo disperse dyes.

The present invention is illustrated in more detail with reference to the following Examples, but is not intended to be limited to the following Examples, in which all parts and percents are by weight unless otherwise indicated.

### Example 1

2,4-Dinitroanisole (70 parts), a water-containing palladium catalyst (3.6 parts, 0.5% palladium on carbon, corresponding to 1.4 parts of dry catalyst) and N,N-dimethylformamide (180 parts) were placed in a 500 ml autoclave, and hydrogenation was conducted at 80° to 85°C. under a hydrogen pressure of 10 to 30 Bars (gauge). Three hours thereafter, hydrogen absorption had ceased. The resulting mixture was filtered to remove the catalyst and the reduction mixture containing 2,4-diaminoanisole (48.3 parts, yield 98.9%) was obtained. The content of 2,4-diaminoanisole was measured by an internal standard method of high speed chromatography.

### Example 2

Example 1 was repeated, except that the hydrogenation was continued for 190 minutes using a water containing palladium catalyst (0.9 parts, 2% Pd on carbon, corresponding to 0.4 part of dry catalyst), to obtain 2,4-diaminoanisole in a yield of 98.8%.

### Example 3

Example 1 was repeated, except that the hydrogenation was continued for 170 minutes using as

the starting material 2,4-dinitrophenetole, to obtain 2,4-diaminophenetole in a yield of 97.6%.

Example 4

Example 2 was repeated, except that the hydrogenation was continued for 155 minutes using as the starting material 2,4-dinitrophenyl methoxyethyl ether, to obtain 2,4-diaminophenyl methoxyethyl ether in a yield of 96.3%.

Example 5

Example 1 was repeated, except that the hydrogenation was continued for 130 minutes using as the starting material 2,4-dinitrodiphenyl ether to obtain 2,4-diaminodiphenyl ether in a yield of 98.3%.

Example 6

2,4-Diaminoanisole (100 parts) was dissolved in N,N-dimethylformamide (600 parts) under a nitrogen atmosphere and then cooled to 10°C. Acetic anhydride (74 parts, i.e. in equimolar ratio to the 2,4-diaminoanisole) was added dropwise to the resulting solution over a period of 2 hours. The mixture was stirred for 30 minutes and thereafter the solvent and by-product acetic acid were distilled off to obtain a concentrate (129 parts).

The composition of the concentrate was as shown below:

| | |
|---|---|
| 2-Amino-4-acetylaminoanisole | 96.0% |
| 2-Acetylamino-4-aminoanisole | less than 0.1% |
| 2,4-Bis(acetylamino)anisole | 1.5% |
| 2,4-Diaminoanisole | 1.5% |
| Others | less than 1.0% |

The concentrate was subjected to simple distillation to collect a fraction (119 parts) at 180° to 195°C./l mmHg. The resulting product had a 2-amino-4-acetylaminoanisole content of 99%, and the over-all yield from the starting 2,4-diaminoanisole was 90.3%.

Comparative Example 1

Acetylation and concentration were carried out each in a manner similar to that of Example 6, except that methanol was used in place of N,N-dimethylformamide as the solvent, whereby there was obtained a concentrate (130 parts) having the following composition:

| | |
|---|---|
| 2-Amino-4-acetylaminoanisole | 79.0% |
| 2,4-Diaminoanisole | 7.4% |
| 2,4-Bis(acetylamino)anisole | 12.1% |
| Others | 1.5% |

Comparative Example 2

Acetylation and concentration were carried out each in a manner similar to that of Example 6, except that acetone was used in place of N,N-dimethylformamide, whereby there was obtained a concentrate (135 parts) having the following composition:

| | |
|---|---|
| 2-Amino-4-acetylaminoanisole | 75.0% |
| 2,4-Diaminoanisole | 5.7% |
| 2,4-Bis(acetylamino)anisole | 18.0% |
| Others | 1.3% |

Comparative Example 3

Acetylation and concentration were carried out each in a manner similar to that of Example 6, except that a water-ethanol mixed solvent (the mixing ratio by weight being 4:1) was used in place of N,N-dimethylformamide, whereby there was obtained a concentrate (131 parts) having the following composition:

| | |
|---|---|
| 2-Amino-4-acetylaminoanisole | 78.2% |
| 2,4-Diaminoanisole | 4.9% |
| 2,4-Bis(acetylamino)anisole | 15.3% |
| Others | 1.6% |

Example 7

2,4-Diaminodiphenyl ether (100 parts) was dissolved in a mixed solvent (600 parts) consisting of N,N-dimethylacetamide (550 parts) and methanol (50 parts) under a nitrogen atmosphere, and then cooled to 5°C. Acetic anhydride (51 parts, i.e. in equimolar ratio to the 2,4-diaminodiphenyl ether) was added dropwise to the resulting solution over a period of 2 hours, and thereafter the mixture was stirred for 30 minutes. The solvent and by-product acetic acid (400 parts in total amount) were distilled off to obtain a concentrate. Subsequently, the concentrate was mixed with water (500 parts), and a 10% aqueous sodium hydroxide solution was added thereto to adjust pH of the mixture to 7. The mixture was stirred for 1 hour to precipitate crystals, which were separated by filtration, washed with water and then dried. Thus, a dried product (119 parts) was obtained, and the composition thereof was as shown below:

| | |
|---|---|
| 2-Amino-4-acetylaminodiphenyl ether | 96.9% |
| 2,4-Diaminodiphenyl ether | 0.7% |
| 2,4-Bis(acetylamino)diphenyl ether | 2.1% |
| Others (unknown matters) | 0.3% |

Comparative Example 4

2,4-Diaminodiphenyl ether (100 parts) was dissolved in a mixture consisting of 35% hydrochloric acid (104 parts) and water (196 parts), and then cooled to 10°C. Acetic anhydride (53 parts, i.e. in a molar ratio of 1.04 to the 2,4-diaminodiphenyl ether) was added dropwise to the resulting solution over a period of 4 hours, during which the temperature was kept at 10°C. or below and 40% aqueous sodium hydroxide solution was added thereto to keep pH of the system from 1.5 to 3.5. Thereafter, the reaction mixture was stirred for 1 hour and 35% hydrochloric acid was added to the reaction mixture to adjust the pH to 1. Sodium chloride (100 parts) was added thereto and the mixture was stirred for another 1 hour to precipitate crystals, which were then separated by filtration, washed with a 20% aqueous sodium chloride solution and dried. A dried product (132 parts) was obtained and the composition thereof was as shown below:

| | |
|---|---|
| 2-Amino-4-acetylaminodiphenyl ether hydrochloride | 71.1% |
| 4-Amino-2-acetylaminodiphenyl ether hydrochloride | 9.3% |
| 2,4-Diaminodiphenyl ether dihydrochloride | 4.7% |
| 2,4-Bis(acetylamino)diphenyl ether | 11.9% |
| Others | 3.0% |

The yield of the desired product was 67.4%.

Example 8

2,4-Diaminophenetole (100 parts) was dissolved in a mixed solvent (600 parts) consisting of N,N-dimethylformamide (550 parts) and water (50 parts) and then cooled to 10°C. Benzoic anhydride (152 parts, i.e. in a molar ratio of 1.02 to the 2,4-diaminophentole) was added thereto over a period of 2 hours. The mixture was thereafter stirred for 30 minutes and then subjected to distillation under reduced pressure to remove the solvent (450 parts). The resulting concentrate was mixed with water (600 parts) under a nitrogen atmosphere, and a 10% aqueous sodium hydroxide solution was added thereto to adjust pH of the mixture to 7. The mixture was further stirred for 1 hour to precipitate crystals, which were then separated by filtration, washed with water and then dried. Thus, a dried product (165 parts) was obtained and the composition thereof was as shown below:

| | |
|---|---|
| 2-Amino-4-benzoylaminophenetole | 95.8% |
| 2,4-Diaminophenetole | 1.3% |
| 2,4-Bis(benzoylamino)phenetole | 2.3% |
| Others (unknown matters) | 0.6% |

The yield of the desired product was 95.1%.

Example 9

2,4-Diamino-2',4'-dichlorodiphenyl ether (100 parts) was dissolved in N,N-dimethylformamide (600 parts) under a nitrogen atmosphere and then cooled to 10°C. Propionic anhydride (49 parts, i.e. in a molar ratio of 1.01 to the 2,4-diamino-2',4'-dichlorodiphenyl ether) was added to the resulting solution over a period of 2 hours. The mixture was stirred for 30 minutes, and then subjected to distillation under reduced pressure to remove the solvent and by-product propionic acid (400 parts in total amount). Thereafter the resulting concentrate was mixed with water (500 parts) and a 10% aqueous sodium hydroxide solution was added thereto to adjust the pH to 7. The mixture was further stirred for 1 hour to precipitate crystals, which were then separated by filtration, washed with water and dried. Thus, a dried product (118 parts) was obtained and the composition thereof was as shown below:

| | |
|---|---|
| 2-Amino-4-propionylamino-2',4'-dichlorodiphenyl ether | 96.2% |
| 2,4-Diamino-2',4'-dichlorodiphenyl ether | 1.3% |
| 2,4-Bis(propionylamino)-2',4'-dichlorodiphenyl ether | 2.1% |
| Others (unknown matters) | 0.4% |

The yield of the desired product was 95.4%.

Example 10

2,4-Dinitroanisole (70 parts), a water-containing palladium catalyst (3.6 parts, 0.5% palladium on carbon, corresponding to 1.4 parts of dry catalyst) and N,N-dimethylformamide (140 parts) were placed in a 500 ml autoclave, and hydrogenation was conducted at 80° to 85°C. under a hydrogen pressure of 10 to 30 kg/cm$^2$ (gauge). Three hours thereafter, hydrogen absorption had ceased. The resulting mixture was filtered to remove the catalyst, and the reduction mixture (214.3 parts) containing 2,4-diaminoanisole (48.2 parts, yield 98.8%) was obtained. The content of 2,4-diaminoanisole was measured by an internal standard method of high speed chromatography.

The resulting reduction mixture was cooled to 10°C. and acetic anhydride (35.6 parts, i.e in equimolar ratio to the 2,4-diaminoanisole) was added dropwise over a period of 2 hours. The mixture was stirred for 30 minutes and then the solvent and the by-product acetic acid were distilled off under reduced pressure to obtain a concentrate (62.2 parts). The composition thereof was as shown below:

| | |
|---|---|
| 2-Amino-4-acetylaminoanisole | 95.8% |
| 2-Acetylamino-4-aminoanisole | less than 0.1% |
| 2,4-Bis(acetylamino)anisole | 1.5% |
| 2,4-Diaminoanisole | 1.2% |
| Others | 1.4% |

The concentrate was subjected to simple distillation under reduced pressure to collect a fraction (57.4 parts) at 180° to 190°C/l mmHg. The 2-amino-4-acetylaminoanisole content in the resulting product was found to be 98.8%. The overall yield on the basis of 2,4-dinitroanisole was 89.1% and the acylation yield on the basis of 2,4-diaminoanisole was 90.2%.

For comparison, the above reduction and acylation procedures were repeated, except that methanol was used in place of N,N-dimethylformamide, to obtain a concentrate (63.2 parts). The composition thereof was as shown below:

| | |
|---|---|
| 2-Amino-4-acetylaminoanisole | 78.2% |
| 2-Acetylamino-4-aminoanisole | 0.8% |
| 2,4-Bis(acetylamino)anisole | 12.9% |
| 2,4-Diaminoanisole | 6.4% |
| Others | 1.7% |

Example 11

In a manner similar to that of Example 10, the reduction and acylation were carried out, except that water (30 parts) was used in addition to N,N-dimethylformamide as the solvent, and the hydrogenation was continued for 195 minutes, to obtain a reduction mixture (244.4 parts) containing 2,4-diaminoanisole (47.2 parts, yield 96.7%), and a concentrate (62.4 parts), respectively. The composition of the concentrate was as shown below:

| | |
|---|---|
| 2-Amino-4-acetylaminoanisole | 95.5% |
| 2-Acetylamino-4-aminoanisole | less than 0.1% |
| 2,4-Bis(acetylamino)anisole | 2.3% |
| 2,4-Diaminoanisole | 0.9% |
| Others | 1.2% |

The concentrate obtained was subjected to simple distillation under reduced pressure to collect a fraction (56.0 parts) at 180° to 195°C./l mmHg. The 2-amino-4-acetylaminoanisole content in the product was found to be 98.6%. The overall yield on the basis of 2,4-dinitroanisole was 86.7%, and the acylation yield on the basis of 2,4-diaminoanisole was 89.7%.

Example 12

In a manner similar to that of Example 11, the reduction was carried out, except that 2,4-dinitrophenetole was used in place of 2,4-dinitroanisole and the hydrogenation was continued for 185 minutes, to obtain a reduction mixture (242.8 parts) containing 2,4-diaminophenetole (48.8 parts, yield 97.3%).

The reduction mixture obtained was cooled to 10°C., and then benzoic anhydride (74.2 parts, i.e. in a molar ratio of 1.02 to the 2,4-diaminophenetole) was added thereto over a period of 2 hours. The mixture was stirred for 30 minutes, and then the solvent (220 parts) was distilled off to obtain a concentrate.

The concentrate was mixed with water (293 parts) and then adjusted to pH 7 using a 10% aqueous sodium hydroxide solution. The resulting mixture was stirred for 1 hour to precipitate crystals, which were then separated by filtration, washed with water and dried. Thus, a dried product (80.5 parts) was obtained, and the composition thereof was as shown below:

| | |
|---|---|
| 2-Amino-4-benzoylaminophenetole | 95.6% |
| 2,4-Diaminophenetole | 0.9% |
| 2,4-Bis(benzoylamino)phenetole | 2.6% |
| Others (unknown matters) | 0.9% |

The overall yield on the basis of 2,4-dinitrophenetole was 92.4% and the acylation yield on the basis of 2,4-diaminophenetole was 95.0%.

Example 13

A reduction mixture (214.3 parts) containing 2,4-diaminoanisole (48.2 parts) obtained in the same manner as in Example 10 was cooled to 10°C., and acetic anhydride (28.4 parts, i.e. in a molar ratio of 0.8 to the 2,4-diaminoanisole) was added dropwise thereto over a period of 2 hours. The mixture was stirred for 30 minutes and then the solvent and the by-product acetic acid were distilled off to obtain a concentrate (60.3 parts). The composition of the concentrate was as shown below:

| | |
|---|---|
| 2-Amino-4-acetylaminoanisole | 83.0% |
| 2-Acetylamino-4-aminoanisole | less than 0.1% |
| 2,4-Bis(acetylamino)anisole | 0.4% |
| 2,4-Diaminoanisole | 15.5% |
| Others | 1.0% |

The concentrate obtained was subjected to simple distillation under reduced pressure to collect a fraction (9.5 parts) having a 2,4-diaminoanisole content of 96% and a fraction (48.0 parts) having a 2-amino-4-acetylaminoanisole content of 99%, at 150° to 160°C./l mmHg and 180° to 195°C./l mmHg, respectively. The recovered 2,4-diaminoanisole corresponded to 18.9% of the whole 2,4-diaminoanisole used for the acetylation, and the acylation yield on the basis of 2,4-diaminoanisole was 75.6%.

Example 14

In a manner similar to that of Example 10, the reduction and acylation were carried out, except that N,N-dimethylacetamide was used in place of N,N-dimethylformamide and the hydrogenation was continued for 185 minutes, to obtain a reduction mixture (213.9 parts) containing 2,4-diaminoanisole (47.9 parts, yield 98.1%), and a concentrate (61.4 parts), respectively. The composition of the concentrate was as shown below:

| | |
|---|---|
| 2-Amino-4-acetylaminoanisole | 95.4% |
| 2-Acetylamino-4-aminoanisole | less than 0.1% |
| 2,4-Bis(acetylamino)anisole | 1.4% |
| 2,4-Diaminoanisole | 1.0% |
| Others | 2.1% |

The concentrate obtained was subjected to simple distillation under reduced pressure to collect a fraction (56.5 parts) at 180° to 195°C./1 mmHg. The 2-amino-4-acetylaminoanisole content in the product was 98.4%. The overall yield on the basis of 2,4-dinitroanisole was 87.3% and the acylation yield on the basis of 2,4-diaminoanisole was 89.0%.

**Claims**

1. A process for producing a 2-amino-4-acylaminophenyl ether of the formula

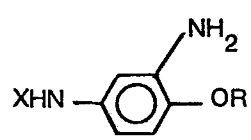

wherein R is $C_1$—$C_9$ alkyl group, a halogen-, $C_1$—$C_3$ alkoxy-, phenyl- or phenoxy-substituted $C_1$—$C_4$ alkyl group or a phenyl group unsubstituted or substituted with one or two substituents selected from halogen atoms, $C_1$—$C_4$ alkyl groups, $C_1$—$C_4$ alkoxy groups and acylamino groups, and X is an acyl group, by contacting a 2,4-diaminophenyl ether of the formula

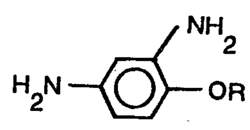

wherein R is as defined above with an acylating agent in a solvent, characterised in that the solvent is selected from N-substituted amides of lower aliphatic acids and mixtures of such N-substituted acid amide with at least one other solvent selected from water, lower alcohols, lower carboxylic acids, ketones, and hydrocarbons, in which the amount of the other solvent is at most 40% by weight of the solvent mixture.

2. A process according to claim 1 characterised in that the acylating agent is an aliphatic or aromatic acid halide or anhydride.

3. A process according to claim 1 or 2 characterised in that the amount of the solvent is 2 to 15 times the weight of the 2,4-diaminophenyl ether.

4. A process according to claim 1, 2 or 3 characterised in that the acylating agent is used in an amount of 0.6 to 1.05 mole per mole of the 2,4-diaminophenyl ether.

5. A process according to any preceding claim characterised in that the contacting is carried out at a temperature of −10° to 40°C.

6. A process according to any preceding claim characterised by dissolving the 2,4-diaminophenyl ether in the solvent under an inert gas atmosphere and then adding the acylating agent thereto with stirring.

7. A process for producing a 2,4-diaminophenyl ether of the formula

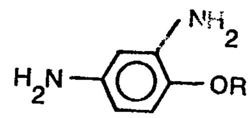

wherein R is as defined in claim 1 by catalytic hydrogenation of a 2,4-dinitrophenyl ether of the formula

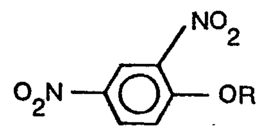

wherein R is as defined above in a solvent, characterised in that the solvent is selected from N-substituted amides of lower aliphatic acids and mixtures of such N-substituted acid amide with at least one other solvent selected from water, lower alcohols, lower carboxylic acids, ketones, and hydrocarbons, in which the amount of the other solvent is at most 30% by weight of the solvent mixture.

8. A process according to claim 7 characterised in that the amount of the solvent is 0.5 to 10 times the weight of the 2,4-dinitrophenyl ether.

9. A process according to claim 7 or 8 characterised in that the hydrogenation is carried out in the presence of a catalyst containing a metal belonging to Group VIII of Mendelejeff's Periodic Table.

10. A process according to claim 9 characterised in that the catalyst contains platinum or palladium in an amount of 0.0005 to 0.1% by weight based on the weight of the 2,4-dinitrophenyl ether.

11. A process according to claim 9 characterised in that the catalyst is palladium carbon or platinum carbon in an amount of 0.1 to 20% by weight based on the weight of the 2,4-dinitrophenyl ether.

12. A process according to claim 9 characterised in that the catalyst is Raney nickel in an amount of 0.1 to 20% by weight based on the weight of the 2,4-dinitrophenyl ether.

13. A process according to any of claims 7 to 12 characterised in that the hydrogenation is carried out at a temperature of 20° to 150°C.

14. A process according to any of claims 7 to 13 characterised in that the hydrogenation is carried out under hydrogen pressure of up to 100 Bars (gauge).

15. A process according to any of claims 1 to 6 characterised in that the 2,4-diaminophenyl ether solution to be contacted with the acylating agent is one obtained from a process according to any of claims 7 to 14.

16. A process according to any preceding claim characterised in that the N-substituted acid amide is selected from N,N-dimethylformamide, N-methylformamide, N,N-dimethylacetamide and N-methylpropionamide.

**Patentansprüche**

1. Verfahren zur Herstellung eines 2-Amino-4-acyl-aminophenylethers der Formel

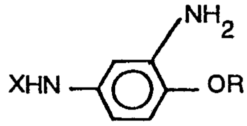

worin R für eine $C_1$—$C_9$-Alkylgruppe, eine Halogenatom, eine $C_1$—$C_3$-Alkoxy-, Phenyl- oder Phenoxy-substituierte $C_1$—$C_4$-Alkylgruppe oder eine nichtsubstituierte Phenylgruppe oder eine Phenylgruppe, substituiert mit einem oder zwei Substituenten, ausgewählt aus Halogenatomen, $C_1$—$C_4$-Alkylgruppen, $C_1$—$C_4$-alkoxygruppen sowie Acylaminogruppen, steht, und X eine Acylgruppe bedeutet, durch Kontaktieren eines 2,4-Diaminophenylethers der Formel

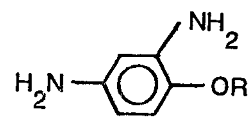

worin R die vorstehend angegebene Bedeutung besitzt, mit einem Acylierungsmittel in einem Lösungsmittel, dadurch gekennzeichnet, daß das eingesetzte Lösungsmittel aus N-substituierten Amiden niedriger aliphatischen Säuren oder Mischungen derartiger N-substituierter Säureamide mit wenigstens einem anderen Lösungsmittel, ausgewählt aus Wasser, niederen Alkoholen, niederen Carbonsäuren, Ketonen und Kohlenwasserstoffen, besteht, wobei die Menge des anderen Lösungsmittels höchsten 40 Gew.-% der Lösungsmittelmischung ausmacht.

2. Verfahren nach Anspruch 1, dadurch gekennzeichnet, daß das eingesetzte Acylierungsmittel ein aliphatisches oder aromatisches Säurehalogenid oder -anhydrid ist.

3. Verfahren nach Anspruch 1 oder 2, dadurch gekennzeichnet, daß die Menge des eingesetzten Lösungsmittels das 2- bis 15-fache des Gewichts des 2,4-Diaminophenylethers ausmacht.

4. Verfahren nach Anspruch 1, 2 oder 3, dadurch gekennzeichnet, daß das Acylierungsmittel in einer Menge von 0,6 bis 1,05 Mol pro Mol des 2,4-Diaminophenylethers verwendet wird.

5. Verfahren nach einem der vorhergehenden Ansprüche, dadurch gekennzeichnet, daß die Kontaktierung bei einer Temperatur von −10 bis +40°C durchgeführt wird.

6. Verfahren nach einem der vorhergehenden Ansprüche, dadurch gekennzeichnet, daß der 2,4-Diaminophenylether in dem Lösungsmittel unter einer inerten Gasatmosphäre aufgelöst und dann das Acylierungsmittel unter Rühren zugesetzt wird.

7. Verfahren zur Herstellung eines 2,4-Diaminophenylethers der Formel

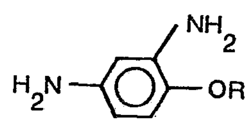

worin R die in Anspruch 1 angegebene Bedeutung besitzt, durch katalytische Hydrierung eines 2,4-Dinitrophenylethers der Formel

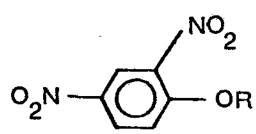

worin R die vorstehend angegebene Bedeutung besitzt, in einem Lösungsmittel, dadurch gekennzeichnet, daß das eingesetzte Lösungsmittel aus N-substituierten Amiden niederer aliphatischer Säuren oder Mischungen derartiger N-substituierter Säureamide mit wenigstens einem anderen Lösungsmittel, ausgewählt aus Wasser, niederen Alkoholen, niederen Carbonsäuren, Ketonen sowie Kohlenwasserstoff, besteht, wobei die Menge des anderen Lösungsmittels höchstens 30 Gew.-% der Lösungsmittelmischung ausmacht.

8. Verfahren nach Anspruch 7, dadurch gekennzeichnet, daß die Menge des eingesetzten Lösungsmittel das 0,5- bis 10-fache des Gewichts des 2,4-Dinitrophenylethers ausmacht.

9. Verfahren nach Anspruch 7 oder 8, dadurch gekennzeichnet, daß die Hydrierung in Gegenwart eines Katalysators durchgeführt wird, der ein Metall enthält, das der Gruppe VIII des Mendeljeff'schen Periodischen Systems der Elemente gehöhrt.

10. Verfahren nach Anspruch 9, dadurch gekennzeichnet, daß der eingesetzte Katalysator Platin oder Palladium in einer Menge von 0,0005 bis 0,1 Gew.-%, bezogen auf das Gewicht des 2,4-Dinitrophenylethers, enthält.

11. Verfahren nach Anspruch 9, dadurch gekennzeichnet, daß der eingesetzte Katalysator aus Palladium/Kohlenstoff oder Platin/Kohlenstoff in einer Menge von 0,1 bis 20 Gew.-%, bezogen auf das Gewicht des 2,4-Dinitrophenylethers, besteht.

12. Verfahren nach Anspruch 9, dadurch gekennzeichnet, daß der eingesetzte Katalysator aus

Raneynickel besteht und in einer Menge von 0,1 bis 20 Gew.-%, bezogen auf das Gewicht des 2,4-Dinitrophenylethers, verwendet wird.

13. Verfahren nach einem der Ansprüche 7 bis 12, dadurch gekennzeichnet, daß die Hydrierung bei einer Temperatur von 20 bis 150°C durchgeführt wird.

14. Verfahren nach einem der Ansprüche 7 bis 13, dadurch gekennzeichnet, daß die Hydrierung unter einem Wasserstoffdruck von bis zu 100 bar (absolut) durchgeführt wird.

15. Verfahren nach einem der Ansprüche 1 bis 6, dadurch gekennzeichnet, daß die 2,4-Diaminophenyletherlösung, die mit dem Acylierungsmittel kontaktiert wird, eine Lösung ist, die nach einem Verfahren gemäß einem der Ansprüche 7 bis 14 erhalten worden ist.

16. Verfahren nach einem der vorhergehenden Ansprüche, dadurch gekennzeichnet, daß das eingesetzte N-substituierte Säureamid aus N,N-Dimethylformamid, N-Methylformamid, N,N-Dimethylacetamid sowie N-Methylpropionamid ausgewählt wird.

**Revendications**

1. Procédé de production d'un 2-amino-4-acylaminophényléther de formule

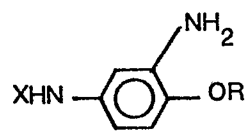

dans laquelle R est un groupe alkyle en $C_1$ à $C_9$, un groupe alkyle en $C_1$ à $C_4$ à substituant halogéno, alkoxy en $C_1$ à $C_3$, phényle ou phénoxy ou un groupe phényle non substitué ou substitué avec un ou deux substituants choisis entre des atomes d'halogènes, des groupes alkyle en $C_1$ à $C_4$, des groupes alkoxy en $C_1$ à $C_4$ et des groupes acylamino, et X est un groupe acyle, par mise en contact d'un 2,4-diaminophényléther de formule

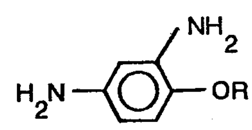

dans laquelle R a la définition donnée ci-dessus, avec un agent acylant dans un solvant, caractérisé en ce que le solvant est choisi entre des amides N-substitués d'acides aliphatiques inférieurs et des mélanges d'un tel amide d'acide N-substitué avec au moins un autre solvant choisi entre l'eau, des alcools inférieurs, des acides carboxyliques inférieurs, des cétones et des hydrocarbures, la quantité de l'autre solvant étant au plus égale à 40% en poids du mélange de solvants.

2. Procédé suivant la revendication 1, caractérisé en ce que l'agent acylant est un halogénure d'acide ou un anhydride d'acide aliphatique ou aromatique.

3. Procédé suivant la revendication 1 ou 2, caractérisé en ce que la quantité de solvant est de 2 à 15 fois le poids du 2,4-diaminophényléther.

4. Procédé suivant la revendication 1, 2 ou 3, caractérisé en ce que l'agent acylant est utilisé en une quantité de 0,6 à 1,05 mole par mole du 2,4-diaminophényléther.

5. Procédé suivant l'une quelconque des revendications précédentes, caractérisé en ce que le contact est effectué à une température de −10 à 40°C.

6. Procédé suivant l'une quelconque des revendications précédentes, caractérisé par la dissolution du 2,4-diaminophényléther dans le solvant sous une atmosphère de gaz inerte, puis l'addition de l'agent acylant à la solution sous agitation.

7. Procédé de production d'un 2,4-diaminophényléther de formule

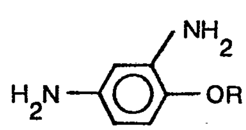

dans laquelle R a la définition donnée dans la revendication 1, par hydrogénation catalytique d'un 2,4-dinitrophényléther de formule

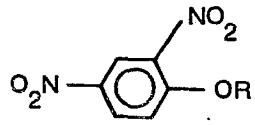

dans laquelle R a la définition donnée ci-dessus, dans un solvant, caractérisé en ce que le solvant est choisi entre des amides N-substitués d'acides aliphatiques inférieurs et des mélanges d'un tel amide d'acide N-substitué avec au moins un autre solvant choisi entre l'eau, des alcools inférieurs, des acides carboxyliques inférieurs, des cétones et des hydrocarbures, la quantité de l'autre solvant étant au plus égale à 30% en poids du mélange de solvants.

8. Procédé suivant la revendication 7, caractérisé en ce que la quantité de solvant est de 0,5 à 10 fois le poids du 2,4-dinitrophényléther.

9. Procédé suivant la revendication 7 ou 8, caractérisé en ce que l'hydrogénation est conduite en présence d'un catalyseur contenant un métal appartenant au Groupe VIII du Tableau Périodique de Mendelejeff.

10. Procédé suivant la revendication 9, caractérisé en ce que le catalyseur contient du platine ou du palladium en une quantité de 0,005 à 0,1% en poids sur la base du poids du 2,4-dinitrophényléther.

11. Procédé suivant la revendication 9, caractérisé en ce que le catalyseur consiste en palladium-carbone ou en platine-carbone en une quantité de 0,1 à 20% en poids sur la base du poids du 2,4-dinitrophényléther.

12. Procédé suivant la revendication 9, caractérisé en ce que le catalyseur consiste en nickel de Raney en une quantité de 0,1 à 20% en poids sur la base du poids du 2,4-dinitrophényléther.

13. Procédé suivant l'une quelconque des revendications 7 à 12, caractérisé en ce que l'hydrogénation est conduite à une température de 20 à 150°C.

14. Procédé suivant l'une quelconque des revendications 7 à 13, caractérisé en ce que l'hydrogénation est conduite sous une pression d'hydrogène s'élevant jusqu'à 100 bars (au manomètre).

15. Procédé suivant l'une quelconque des revendications 1 à 6, caractérisé en ce que la solution de 2,4-diaminophényléther devant être mise en contact avec l'agent acylant est une solution obtenue par un procédé suivant l'une quelconque des revendications 7 à 14.

16. Procédé suivant l'une quelconque des revendications précédentes, caractérisé en ce que l'amide d'acide N-substitué est choisi entre le N,N-diméthylformamide, le N-méthylformamide, le N,N-diméthylacétamide et le N-méthylpropionamide.